# EUROPEAN PATENT SPECIFICATION

(11) **EP 3 270 764 B1**
(45) Date of publication and mention of the grant of the patent: **30.08.2023**
(21) Application number: 16710249.0
(22) Date of filing: 17.03.2016
(51) Int. Cl.: B60K 28/06, A61B 5/18, G08G 1/16, B60Q 9/00, G08B 21/06

(54) **SITUATIONAL AWARENESS MONITORING METHOD AND APPARATUS**
SITUATIONSBEWUSSTES ÜBERWACHUNGSVERFAHREN UND VORRICHTUNG
PROCÉDÉ ET APPAREIL DE SURVEILLANCE DE LA CONSCIENCE DE LA SITUATION

(30) Priority: 18.03.2015 GB 201504600
(43) Date of publication of application: 24.01.2018
(73) Proprietor: Jaguar Land Rover Limited, Coventry, Warwickshire CV3 4LF (GB)
(72) Inventor: DAWSON, Mark, Coventry Warwickshire CV3 4LF (GB); O'DONOGHUE, Jim, Coventry Warwickshire CV3 4LF (GB)
(74) Representative: Jaguar Land Rover Patents Department
(86) International application number: PCT/EP2016/055892
(87) International publication number: WO 2016/146790

(56) References cited:
- US-A1- 2014 125 474
- US-A1- 2014 210 978

## Description

### TECHNICAL FIELD

The present disclosure relates to a situational awareness monitoring method and apparatus. Aspects and embodiments of the invention relate to a method of monitoring situational awareness of a vehicle driver, to an apparatus and to a vehicle.

### BACKGROUND

US 2014/125474 discloses a pre-collision safety warning system configured to calculate an EOR (eyes off-the-road) parameter based on gaze location and eye fixation duration, proportional to driver's perception of external hazard, comprising external sensors for tracking external events and objects, such as a braking vehicle, a hazard level being estimated based on the external events and objects, combined with the driver's hazard attentiveness.

It is known to measure head and eye movements to determine a gaze direction of ar individual. The gaze direction can be used to determine what the individual is looking at. The accuracy with which the determination can be performed is dependent on the monitoring accuracy of the gaze direction. A relatively coarse monitoring accuracy is sufficient when operating over short distances, but higher monitoring accuracy is required to operate over longer distances. In the context of monitoring a driver in a vehicle, a coarse monitoring accuracy can be used to determine what the driver is looking at within an occupant compartment (cabin). For example, a monitoring accuracy of between 5° and 10° may prove adequate to determine where the driver is looking within the occupant compartment. However, over longer distances this level of accuracy may prove inadequate. By way of example, a system accurate to within 7° can be offset laterally by up to 13 metres at a range of 50 metres. A higher level of accuracy would be required accurately to track external objects, but the associated costs are prohibitive.

It is against this background that the present invention has been conceived. At least in certain embodiments, the present invention seeks to overcome or ameliorate at least some of the shortcomings associated with prior art devices.

### SUMMARY OF THE INVENTION

Aspects of the present invention relate to a situational awareness monitoring apparatus; a method of monitoring situational awareness of a vehicle driver; and a vehicle.

According to an aspect of the invention, there is provided an apparatus for a vehicle comprising a processor configured to:
receive eye tracking information from an eye tracking apparatus configured to track driver eye movements;
analyse the eye tracking information to generate an eye tracking signature, wherein the eye tracking signature comprises angular velocity;
receive object tracking information from an object tracking apparatus configured to track one or more external objects;
analyse the object tracking information to generate an object tracking signature, wherein the object tracking signature comprises angular velocity;
correlate the angular velocity of the eye tracking signature with the angular velocity of the object tracking signature to determine when the driver eye movement corresponds to relative movement of the external object; and
adjust a sensitivity level of a vehicle system in dependence on the determined correlation.

The apparatus of some embodiments can monitor the situational awareness of the driver to determine if the driver is aware of one or more objects around the vehicle. By comparing the eye movements of the driver with the movement of the object relative to the vehicle, the processor can determine if the driver is aware of an object. The apparatus of some embodiments can determine where and how the driver is looking to monitor situational awareness of the driver. At least in certain embodiments, the apparatus can determine whether the driver is aware of one or more object external to the car. If the eye movements at least substantially match movement of the one or more object, the processor can determine that the driver is aware of the object. If the eye movements do not at least substantially match the object movement, the processor can determine that the driver is not aware of the object. The apparatus may thereby determine when the driver is aware of one or more object external to the vehicle.

The eye tracking information may comprise gaze direction and/or eye movement information. The eye tracking signature may comprise a saccade pattern or signature. The saccade pattern represents eye movements. The processor may correlate the eye saccade patterns with movement of one or more external object. The saccade patterns could correspond to the driver tracking a single object continuously, or tracking a plurality of objects sequentially. The one or more objects may be tracked continuously by the object tracking apparatus.

A filter may be applied to the eye tracking information to remove fluctuations in the detected eye movement.

The eye tracking signature may comprise one or more of the following: angular acceleration; direction; velocity; and acceleration. The eye tracking information can comprise gaze direction representing a direction in which the driver is looking. For example, gaze direction can be used as a coarse filter so that the processor does not need to compute all detected objects.

The object tracking signature may define movement of the external object relative to the vehicle.

The object tracking signature may comprise one or more of the following: angular acceleration; direction; velocity; and acceleration. The velocity may be absolute velocity or relative velocity. The acceleration may be absolute acceleration or relative acceleration.

By comparing angular velocity, the correlation may be performed using gaze trackers having a lower accuracy than would be required to determine where the driver was looking based solely on gaze direction. This is particularly significant over larger distances where a small angular offset may equate to a relatively large distance. It will be appreciated that the processor may optionally utilise gaze direction in combination with the angular velocity of the eye tracking signature.

The processor may be configured to output a control signal in dependence on the determined correlation between the eye tracking information and the object tracking information. A positive control signal may be output when the driver eye movement at least substantially corresponds to relative movement of the external object. A negative control signal may be output when the driver's eye movement does not at least substantially correspond to relative movement of the external object. The control signal could indicate a measure of the strength of the correlation between the eye tracking information with the object tracking information. For example, the control signal could indicate the correlation as a percentage, ranging from 0% indicating no correlation and 100% indicating perfect correlation.

The object tracking information may comprise a first signal received from at least one first sensor. The at least one first sensor may be in the form of one or more of the following: an optical sensor; an infrared sensor; an ultrasonic sensor; and a radar sensor. The at least one first sensor may be outwardly facing.

The eye tracking information may comprise a second signal received from at least one second sensor. The at least one second sensor may be in the form of an optical sensor and/or an infrared sensor. An infrared illuminator may be used to highlight a pupil in the driver's eye to facilitate identification in optical sensor data. The at least one second sensor may be an optical camera. The at least one second sensor is driver-facing and may be directed inwardly into an occupant cabin.

According to a further aspect of the present invention there is provided a situational awareness monitoring apparatus as described herein in combination with said object tracking apparatus and/or said eye tracking apparatus.

According to a still further aspect of the present invention there is provided a vehicle comprising situational awareness monitoring apparatus as described herein.

According to a further aspect of the present invention there is provided a computerimplemented method of monitoring situational awareness of a vehicle driver, the method comprising:
receiving eye tracking information from an eye tracking apparatus configured to track driver eye movements;
analysing the eye tracking information to generate an eye tracking signature, wherein the eye tracking signature comprises angular velocity;
receiving object tracking information from an object tracking apparatus configured to track one or more external objects;
analysing the object tracking information to generate an object tracking signature, wherein the object tracking signature comprises angular velocity;
correlating the angular velocity of the eye tracking signature with the angular velocity of the object tracking signature to determine when the driver eye movement corresponds to relative movement of the external object; and
adjusting a sensitivity level of a vehicle system in dependence on the determined correlation.

The eye tracking information may comprise gaze direction and/or eye movement information. The eye tracking signature may comprise a saccade pattern or signature. The saccade pattern represents the eye movements. A filter may be applied to the eye tracking information to remove fluctuations in the detected eye movement.

The eye tracking signature may comprise one or more of the following: angular acceleration; direction; velocity; and acceleration.

The object tracking signature may define movement of the external object relative to the vehicle. The object tracking signature may comprise one or more of the following: angular acceleration; direction; velocity; and acceleration. The velocity may be absolute velocity or relative velocity. The acceleration may be absolute acceleration or relative acceleration.

The method may comprise outputting a control signal in dependence on the determined correlation between the eye tracking information with the object tracking information. A positive control signal may be output when the driver eye movement at least substantially corresponds to relative movement of the external object. A negative control signal may be output when the driver eye movement does not at least substantially correspond to relative movement of the external object. The control signal could indicate a measure of the strength of the correlation between the eye tracking information with the object tracking information. For example, the control signal could indicate the correlation as a percentage, ranging from 0% indicating no correlation and 100% indicating perfect correlation.

The object tracking information may comprise a first signal received from at least one first sensor. The at least one first sensor may be in the form of one or more of the following: an optical sensor; an infrared sensor; an ultrasonic sensor; a radar sensor. The at least one first sensor may be outwardly facing.

The eye tracking information may comprise a second signal received from at least one second sensor. The at least one second sensor may be in the form of one or more of the following: an optical sensor or an infrared sensor. The at least one second sensor may be optical camera. The at least one second sensor is driver-facing and may be directed inwardly into an occupant cabin.

The term saccade is used herein to refer a rapid movement of an eye, head or other part of the body. Saccades are quick, simultaneous movements of both eyes in the same direction. Within the scope of this application it is expressly intended that the various aspects, embodiments, examples and alternatives set out in the preceding paragraphs, in the claims and/or in the following description and drawings, and in particular the individual features thereof, may be taken independently or in any combination. That is, all embodiments and/or features of any embodiment can be combined in any way and/or combination, unless such features are incompatible.

### BRIEF DESCRIPTION OF THE DRAWINGS

One or more embodiments of the present invention will now be described, by way of example only, with reference to the accompanying figures, in which:
Figure 1 shows a schematic overview of a vehicle incorporating a situational awareness monitoring apparatus in accordance with an embodiment of the present invention;
Figure 2 shows a schematic representation of the situational awareness monitoring apparatus shown in Figure 1;
Figure 3 shows a graphical representation of a typical situation to be monitored by the situational awareness monitoring apparatus; and
Figure 4 shows a graphical representation of the imaging areas analysed by the situational awareness monitoring apparatus.

### DETAILED DESCRIPTION

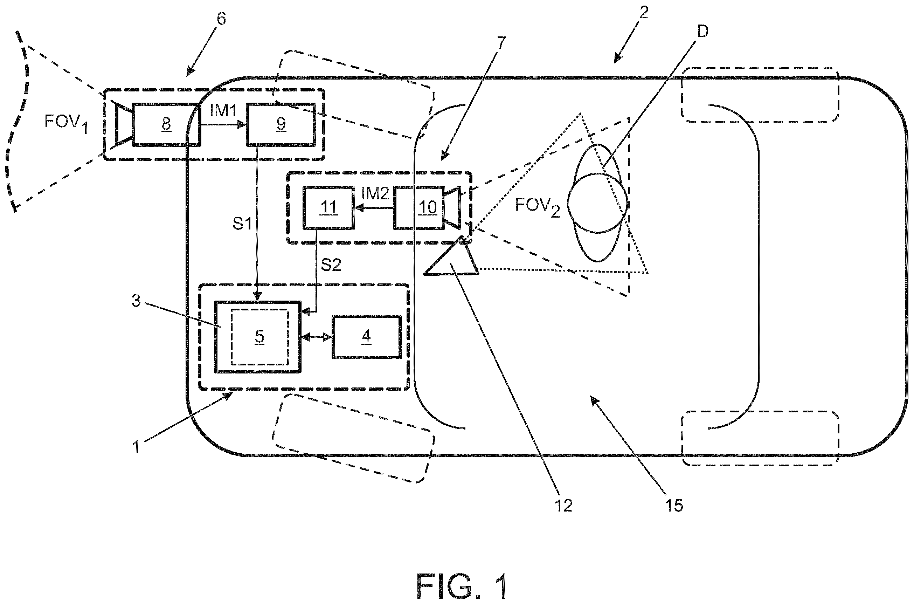

A situational awareness monitoring apparatus 1 in accordance with an embodiment of the present invention will now be described with reference to the accompanying Figures 1 to 4. The situational awareness monitoring apparatus 1 is intended for use in a vehicle 2 to monitor situational awareness of a driver D of the vehicle 2. The vehicle 2 in the present embodiment is an automobile, but the invention is not limited in this respect and the situational awareness monitoring apparatus 1 could be implemented in other vehicle types.

The situational awareness monitoring apparatus 1 comprises an electronic processor 3 coupled to system memory 4. A set of operating instructions are stored in the system memory 4 and are accessible to the electronic processor 3. When executed, the operating instructions cause the electronic processor 3 to operate in accordance with the methods described herein. The electronic processor 3 is configured to implement a comparator module 5 for comparing tracking signatures, as described herein.

The electronic processor 3 is connected to an external object tracking apparatus 6 configured to detect and track one or more object O external to the vehicle 2 with respect to time; and an eye tracking apparatus 7 configured to track eye movements of a driver D of the vehicle 2. The external object tracking apparatus 6 outputs object tracking information S1 to the electronic processor 3; and the eye tracking apparatus 7 outputs are tracking information S2 to the electronic processor 3. The external object tracking apparatus 6 in the present embodiment comprises a first optical camera 8 and a first image processor 9. The eye tracking apparatus 7 in the present embodiment comprises a second optical camera 10, a second image processor 11 and an infrared illuminator 12. It will be appreciated that the external object tracking apparatus 6 could comprise a different type of sensor, such as a radar sensor, an ultrasonic sensor or an infrared sensor. Equally, the eye tracking apparatus 7 could comprise a different type of sensor, such as an infrared sensor.

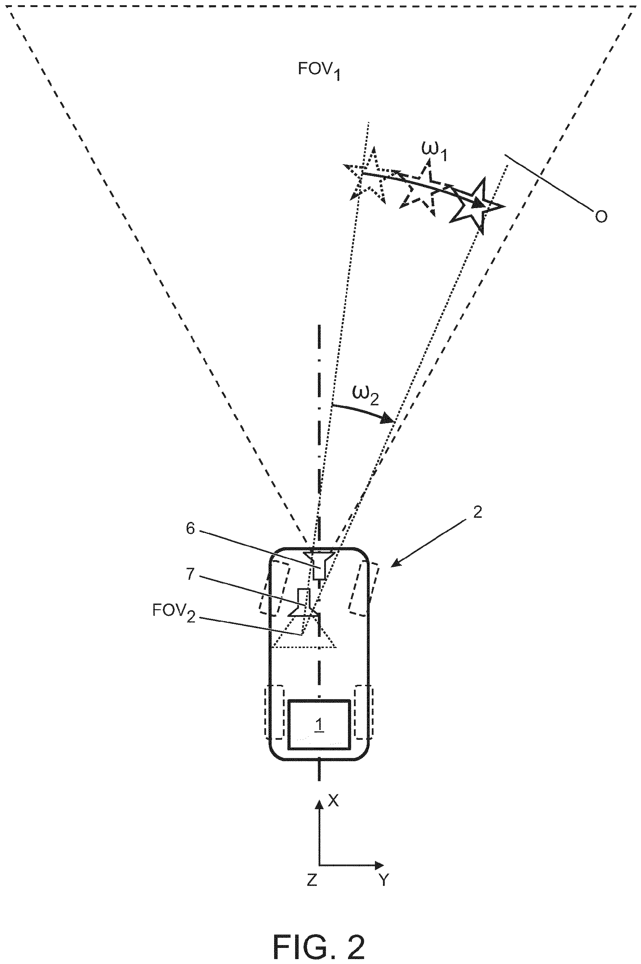

As shown schematically in Figure 2, the first optical camera 8 has a first field of view FOV1 in front of the vehicle 2. The first optical camera 8 outputs first image data IM1 to the first image processor 9 which is configured to analyse the image data to detect and track one or more object O within the first field of view FOV1. The first image processor 9 analyses said first image data IM1 and generates said object tracking information S1 which defines the position of said one or more object O in relation to the vehicle 2. The object tracking information S1 can, for example, define the coordinates of each identified object O relative to a fixed datum point in the vehicle 2. In the illustrated arrangement, the first optical camera 8 is forward-facing and is oriented along a longitudinal axis X of the vehicle 2. The external object tracking apparatus 6 could comprise an array of sensors arranged to provide coverage in a larger area external to the vehicle 2.

The second optical camera 10 is a driver-facing camera, for example disposed in a rear view mirror 13 mounted to a front windshield 14 of the vehicle 2. The second optical camera 10 has a second field of view FOV2 directed inwardly into an occupant compartment (cabin) 15. The infrared illuminator 12 is operable to illuminate the driver's eyes to facilitate detection and tracking of the pupils. The second optical camera 10 outputs second image data IM2 to the second image processor 11. The second image processor 11 analyses the second image data IM2 to identify and track movement of one or both of the driver's eyes. The second image processor 11 determines the position of each eye and a gaze direction for both eyes together. The eye tracking information S2 generated by the second image processor 11 defines a reference point for each eye; and a gaze direction for both eyes. The reference point can be static, for example predefined in dependence on a typical position of the driver's head; or can be dynamic, for example calculated in dependence on a determined head pose of the driver D. The eye tracking information S2 has sufficient resolution to enable determination of saccade eye patterns which correspond to tracking of an object. The eye tracking information S2 could optionally also comprise a depth measurement based on eye position. The second optical camera 10 can optionally also be configured to track driver head pose.

The electronic processor 3 receives the object tracking information S1 and the eye tracking information S2 from the external object tracking apparatus 6 and the eye tracking apparatus 7 respectively. The electronic processor 3 analyses the object tracking information S1 to generate an object tracking signature. The object tracking signature comprises a first angular velocity ω₁ (radians per second) of the object O; and optionally a first angular acceleration ω̇₁ (radians per second per second). The object tracking signature can optionally comprise a first vector defined in three-dimensional space and representing the direction and speed of the object O in relation to the vehicle 2. The electronic processor 3 analyses the eye tracking information S2 to remove erratic fluctuations ("wobble"), for example by applying a filter to the eye tracking information S2 to remove fluctuations having a frequency greater than or less than a predefined threshold. In dependence on the eye tracking information S2, the electronic processor 3 generates an eye tracking signature comprising a second angular velocity ω₂ (radians per second);. The eye tracking signature may represent the saccade movement patterns of the driver's eyes. The eye tracking signature could optionally include a second angular acceleration ω̇₂ (radians per second per second). In this arrangement the eye tracking signature could comprise a second vector defined in three-dimensional space and representing the direction and speed of the movement of the driver's eyes. The first and second angular velocities ω₁, ω₂ and, optionally, the first and second angular accelerations ω̇₁, ω̇₂, can each be defined about one or more axis, for example about a longitudinal axis (X) and/or a transverse axis (Y) and/or a vertical axis (X). The object tracking signature and the eye tracking signature can be defined with reference to a common reference point, such as the reference point identified for the driver's eyes. If the object tracking signature and the eye tracking signature are defined with reference to different reference points, the electronic processor 3 can apply a transform to facilitate comparison.

The electronic processor 3 is configured to correlate the eye tracking signature to the object tracking signature to determine if the driver D is tracking an object O identified by the external object tracking apparatus 6. In particular, the comparator module 5 compares the eye tracking signature and the object tracking signature. The electronic processor 3 compares the first and second angular velocities ω₁, ω₂. Optionally, in addition, the electronic processor can, for example, compare the directions of the first and second vectors. The system memory 4 stores threshold levels for angular velocity ω₁, ω₂, and, optionally the direction. If the comparison shows that the angular velocities are the same or within the defined threshold(s), the electronic processor 3 determines that the driver is tracking the object O. The electronic processor 3 is configured to perform this analysis at least substantially within real time.

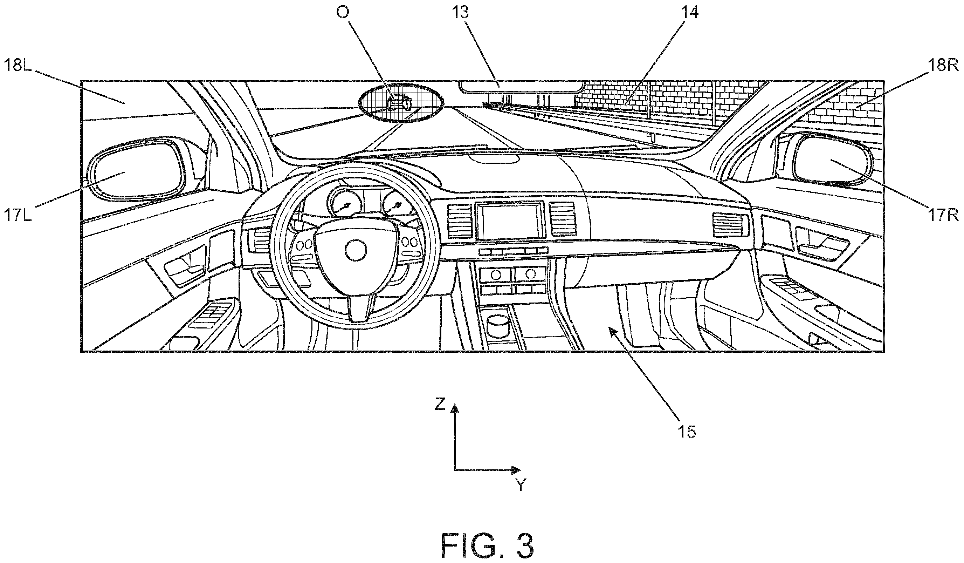

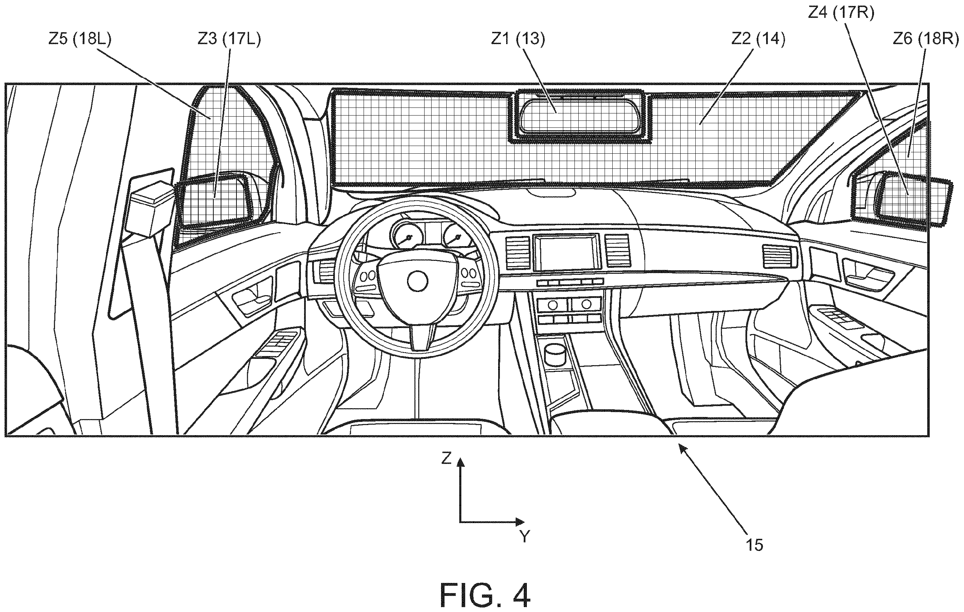

The operation of the situational awareness monitoring apparatus 1 will now be described with reference to Figures 3 and 4. A graphical representation of the occupant compartment 15 is shown in Figure 3. A typical scenario is illustrated in which an object O in the form of another vehicle is visible through the front windshield 14. The object O is within the first field of view FOV1 of the first optical camera 8 and is detected by the external object tracking apparatus 6.

The external object tracking apparatus 6 monitors the position of the object O and generates object tracking information. The electronic processor 3 generates an object signature in dependence on the object tracking information received from the external object tracking apparatus 6. The eye tracking apparatus 7 tracks the gaze direction of the driver's eyes and outputs eye tracking information. The electronic processor 3 generates an eye tracking signature in dependence on the eye tracking information received from the eye tracking apparatus 7. A comparison of the object tracking signature and the eye tracking signature is performed to determine if there is a correlation therebetween. If a correlation is identified between the object tracking signature and the eye tracking signature, the electronic processor 3 determines that the driver D is aware of the object O. In dependence on the strength of the correlation, the electronic processor 3 can also estimate whether the driver D has been able to classify the object O, for example if they have followed its movement for a predetermined period of time (for example a time period of 120ms). It will be appreciated that this technique can be applied to a plurality of objects O identified by the external object tracking apparatus 6.

The electronic processor 3 can output a control signal in dependence on the determined correlation between the object tracking signature and the eye tracking signature. If the correlation is strong (i.e. the electronic processor 3 determines that the eye tracking signature corresponds to the object tracking signature), a positive control signal can be output to indicate that the driver D is aware of the object O. If the correlation is weak (i.e. the electronic processor 3 determines that the eye tracking signature does not correspond to the object tracking signature), a negative control signal can be output to indicate that the driver D is not aware of the object O. The control signal thereby provides an indication of driver situational awareness. The situational awareness monitoring apparatus 1 can be coupled to other vehicle systems operable in dependence on the control signal. For example, the situational awareness monitoring apparatus 1 can be connected to a notification system configured to generate a notification, such as a visual, audio or haptic signal, in dependence on a negative control signal.

A sensitivity level of a vehicle system is adjusted in dependence on the determined correlation. For example, a sensitivity level of a vehicle safety system could be adjusted in dependence on the determined correlation.Optionally, in addition, one or more vehicle systems could be controlled in dependence on the control signal, for example activation or de-activation of an automated vehicle system could be inhibited if the situational awareness monitoring apparatus 1 determines that the driver D is not sufficiently aware of the current situation.

The operation of the situational awareness monitoring apparatus 1 has been described with reference to an object O visible through the front windshield 14. The situational awareness monitoring apparatus 1 is not limited in this respect and can be used in respect of an object O visible in one or more of the following: the rear view mirror 13, the front windshield 14, a left side mirror 17L, a right side mirror 17R, a left side window 18L, and a right side window 18R. As shown in Figure 4, a plurality of operating zones Z1-6 can be defined in the system memory 4, for example in a virtual model of the vehicle 2. The operating zones Z1-6 correspond respectively to the rear view mirror 13, the front windshield 14, the left and right side mirrors 17L, 17R, and the left and right side windows 18L, 18R. The comparator module 5 can modify the comparison of the eye tracking signature with the object tracking signature depending on the location of the object O. For example, if the driver D is looking in the rear view mirror 13, the eye tracking signature is correlated with an object tracking signature generated in dependence on a rear-facing camera. The electronic processor 3 is configured to apply appropriate transforms and/or scaling functions to enable comparison of the object tracking signature and the eye tracking signature. This approach can be applied for the left and right side mirrors 17L, 17R; and the left and right side windows 18L, 18R.

It will be appreciated that various changes and modifications can be made to the embodiment described herein without departing from the scope of the present application.

## Claims

1. An apparatus (1) for a vehicle (2) comprising a processor (5) configured to:
receive eye tracking information from an eye tracking apparatus (7) configured to track driver eye movements;
analyse the eye tracking information to generate an eye tracking signature, wherein the eye tracking signature comprises angular velocity;
receive object tracking information from an object tracking apparatus (6) configured to track one or more external objects;
analyse the object tracking information to generate an object tracking signature, wherein the object tracking signature comprises angular velocity;
correlate the angular velocity of the eye tracking signature with the angular velocity of the object tracking signature to determine when the driver eye movement corresponds to relative movement of the external object; and
adjust a sensitivity level of a vehicle system in dependence on the determined correlation.

2. An apparatus as claimed in claim 1, wherein the eye tracking signature comprises a saccade pattern.

3. An apparatus as claimed in any preceding claim, wherein the eye tracking signature comprises one or more of the following: angular acceleration, velocity; and acceleration.

4. An apparatus as claimed in any preceding claim, wherein the object tracking signature comprises one or more of the following: angular acceleration, velocity; and acceleration.

5. An apparatus as claimed in any one of the preceding claims, wherein the processor is configured to output a control signal in dependence on the determined correlation between the eye tracking signature with the object tracking signature.

6. An apparatus as claimed in any one of the preceding claims, wherein the object tracking information comprises a first signal received from at least one first sensor.

7. An apparatus as claimed in any one of the preceding claims, wherein the eye tracking information comprises a second signal received from at least one second sensor.

8. An apparatus as claimed in any one of the preceding claims in combination with said object tracking apparatus and/or said eye tracking apparatus.

9. A vehicle (2) comprising an apparatus (1) as claimed in any one of the preceding claims.

10. A computer-implemented method of monitoring situational awareness of a vehicle (2) driver, the method comprising:
receiving eye tracking information from an eye tracking apparatus (7) configured to track driver eye movements;
analysing the eye tracking information to generate an eye tracking signature, wherein the eye tracking signature comprises angular velocity;
receiving object tracking information from an object tracking apparatus (6) configured to track one or more external objects;
analysing the object tracking information to generate an object tracking signature, wherein the object tracking signature comprises angular velocity;
correlating the angular velocity of the eye tracking signature with the angular velocity of the object tracking signature to determine when the driver eye movement corresponds to relative movement of the external object; and
adjusting a sensitivity level of a vehicle system in dependence on the determined correlation.

11. A method as claimed in claim 10, wherein the eye tracking signature comprises a saccade pattern.

12. A method as claimed in claim 10 or claim 11, wherein the eye tracking signature and/or the object tracking signature comprises one or more of the following: angular acceleration, velocity; and acceleration.

13. A method as claimed in any one of claims 10 to 12 comprising outputting a control signal in dependence on the determined correlation between the eye tracking signature with the object tracking signature.

14. A method as claimed in any one of claims 10 to 13, wherein the object tracking information comprises a first signal received from at least one first sensor.

15. A method as claimed in any one of claims 10 to 14, wherein the eye tracking information comprises a second signal received from at least one second sensor.

## Patentansprüche

1. Vorrichtung (1) für ein Fahrzeug (2), umfassend einen Prozessor (5), der konfiguriert ist zum:
Empfangen von Augenverfolgungsinformationen von einer Augenverfolgungsvorrichtung (7), die konfiguriert ist, um Augenbewegungen eines Fahrers zu verfolgen;
Analysieren der Augenverfolgungsinformationen, um eine Augenverfolgungssignatur zu erzeugen, wobei die Augenverfolgungssignatur eine Winkelgeschwindigkeit umfasst;
Empfangen von Objektverfolgungsinformationen von einer Objektverfolgungsvorrichtung (6), die konfiguriert ist, um ein oder mehrere externe Objekte zu verfolgen;
Analysieren der Objektverfolgungsinformationen, um eine Objektverfolgungssignatur zu erzeugen, wobei die Objektverfolgungssignatur die Winkelgeschwindigkeit umfasst;
Korrelieren der Winkelgeschwindigkeit der Augenverfolgungssignatur mit der Winkelgeschwindigkeit der Objektverfolgungssignatur, um zu bestimmen, wann die Augenbewegung des Fahrers einer relativen Bewegung des externen Objekts entspricht; und
Anpassen einer Empfindlichkeitsstufe eines Fahrzeugsystems in Abhängigkeit von der bestimmten Korrelation.

2. Vorrichtung nach Anspruch 1, wobei die Augenverfolgungssignatur ein Sakkadenmuster umfasst.

3. Vorrichtung nach einem der vorstehenden Ansprüche, wobei die Augenverfolgungssignatur eine oder mehrere der Folgenden umfasst: Winkelbeschleunigung, Geschwindigkeit; und Beschleunigung.

4. Vorrichtung nach einem der vorstehenden Ansprüche, wobei die Objektverfolgungssignatur eine oder mehrere der Folgenden umfasst: Winkelbeschleunigung, Geschwindigkeit; und Beschleunigung.

5. Vorrichtung nach einem der vorstehenden Ansprüche, wobei der Prozessor konfiguriert ist, um ein Steuersignal in Abhängigkeit von der bestimmten Korrelation zwischen der Augenverfolgungssignatur mit der Objektverfolgungssignatur auszugeben.

6. Vorrichtung nach einem der vorstehenden Ansprüche, wobei die Objektverfolgungsinformationen ein erstes Signal umfassen, das von mindestens einem ersten Sensor empfangen wird.

7. Vorrichtung nach einem der vorstehenden Ansprüche, wobei die Augenverfolgungsinformationen ein zweites Signal umfassen, das von mindestens einem zweiten Sensor empfangen wird.

8. Vorrichtung nach einem der vorstehenden Ansprüche in Kombination mit der Objektverfolgungsvorrichtung und/oder der Augenverfolgungsvorrichtung.

9. Fahrzeug (2), umfassend die Vorrichtung (1) nach einem der vorstehenden Ansprüche.

10. Computerimplementiertes Verfahren zum Überwachen von einem Situationsbewusstsein eines Fahrers des Fahrzeugs (2), das Verfahren umfassend:
Empfangen von Augenverfolgungsinformationen von einer Augenverfolgungsvorrichtung (7), die konfiguriert ist, um Augenbewegungen des Fahrers zu verfolgen;
Analysieren der Augenverfolgungsinformationen, um eine Augenverfolgungssignatur zu erzeugen, wobei die Augenverfolgungssignatur die Winkelgeschwindigkeit umfasst;
Empfangen von Objektverfolgungsinformationen von einer Objektverfolgungsvorrichtung (6), die konfiguriert ist, um ein oder mehrere externe Objekte zu verfolgen;
Analysieren der Objektverfolgungsinformationen, um eine Objektverfolgungssignatur zu erzeugen, wobei die Objektverfolgungssignatur die Winkelgeschwindigkeit umfasst;
Korrelieren der Winkelgeschwindigkeit der Augenverfolgungssignatur mit der Winkelgeschwindigkeit der Objektverfolgungssignatur, um zu bestimmen, wann die Augenbewegung des Fahrers der relativen Bewegung des externen Objekts entspricht; und
Anpassen einer Empfindlichkeitsstufe eines Fahrzeugsystems in Abhängigkeit von der bestimmten Korrelation.

11. Verfahren nach Anspruch 10, wobei die Augenverfolgungssignatur ein Sakkadenmuster umfasst.

12. Verfahren nach Anspruch 10 oder 11, wobei die Augenverfolgungssignatur und/oder die Objektverfolgungssignatur eine oder mehrere der Folgenden umfasst: Winkelbeschleunigung, Geschwindigkeit; und Beschleunigung.

13. Verfahren nach einem der Ansprüche 10 bis 12, umfassend das Ausgeben eines Steuersignals in Abhängigkeit von der bestimmten Korrelation zwischen der Augenverfolgungssignatur mit der Objektverfolgungssignatur.

14. Verfahren nach einem der Ansprüche 10 bis 13, wobei die Objektverfolgungsinformationen ein erstes Signal umfassen, das von mindestens einem ersten Sensor empfangen wird.

15. Verfahren nach einem der Ansprüche 10 bis 14, wobei die Augenverfolgungsinformationen ein zweites Signal umfassen, das von mindestens einem zweiten Sensor empfangen wird.

## Revendications

1. Appareil (1) pour un véhicule (2) comprenant un processeur (5) configuré pour :
recevoir des informations de suivi oculaire d'un appareil de suivi oculaire (7) configuré pour suivre des mouvements oculaires de conducteur ;
analyser les informations de suivi oculaire pour générer une signature de suivi oculaire, dans lequel la signature de suivi oculaire comprend une vitesse angulaire ;
recevoir des informations de suivi d'objet en provenance d'un appareil de suivi d'objet (6) configuré pour suivre un ou plusieurs objets externes ;
analyser les informations de suivi d'objet pour générer une signature de suivi d'objet, dans lequel la signature de suivi d'objet comprend une vitesse angulaire ;
corréler la vitesse angulaire de la signature de suivi oculaire avec la vitesse angulaire de la signature de suivi d'objet pour déterminer quand le mouvement de l'oeil du conducteur correspond à un mouvement relatif de l'objet externe ; et
ajuster un niveau de sensibilité d'un système de véhicule en fonction de la corrélation déterminée.

2. Appareil selon la revendication 1, dans lequel la signature de suivi oculaire comprend un motif saccadé.

3. Appareil selon l'une quelconque revendication précédente, dans lequel la signature de suivi oculaire comprend une ou plusieurs des éléments suivants : accélération angulaire, vitesse, et accélération.

4. Appareil selon l'une quelconque des revendications précédentes, dans lequel la signature de suivi d'objet comprend une ou plusieurs des éléments suivants : accélération angulaire, vitesse ; et accélération.

5. Appareil selon l'une quelconque des revendications précédentes, dans lequel le processeur est configuré pour délivrer un signal de commande en fonction de la corrélation déterminée entre la signature de suivi oculaire avec la signature de suivi d'objet.

6. Appareil selon l'une quelconque des revendications précédentes, dans lequel les informations de suivi d'objet comprennent un premier signal reçu à partir d'au moins un premier capteur.

7. Appareil selon l'une quelconque des revendications précédentes, dans lequel les informations de suivi oculaire comprennent un second signal reçu d'au moins un second capteur.

8. Appareil selon l'une quelconque des revendications précédentes en combinaison avec ledit appareil de suivi d'objet et/ou ledit appareil de suivi oculaire.

9. Véhicule (2) comprenant un appareil (1) selon l'une quelconque des revendications précédentes.

10. Procédé mis en oeuvre par ordinateur de surveillance de conscience de situation d'un conducteur de véhicule (2), le procédé comprenant :
la réception d'informations de suivi oculaire d'un appareil de suivi oculaire (7) configuré pour suivre des mouvements oculaires de conducteur ;
l'analyse des informations de suivi oculaire pour générer une signature de suivi oculaire, dans lequel la signature de suivi oculaire comprend une vitesse angulaire ;
la réception d'informations de suivi d'objet à partir d'un appareil de suivi d'objet (6) configuré pour suivre un ou plusieurs objets externes ;
l'analyse des informations de suivi d'objet pour générer une signature de suivi d'objet, dans lequel la signature de suivi d'objet comprend une vitesse angulaire ;
la corrélation de la vitesse angulaire de la signature de suivi oculaire avec la vitesse angulaire de la signature de suivi d'objet pour déterminer quand le mouvement de l'œil du conducteur correspond à un mouvement relatif de l'objet externe ; et
l'ajustement d'un niveau de sensibilité d'un système de véhicule en fonction de la corrélation déterminée.

11. Procédé selon la revendication 10, dans lequel la signature de suivi oculaire comprend un motif saccadé.

12. Procédé selon la revendication 10 ou la revendication 11, dans lequel la signature de suivi oculaire et/ou la signature de suivi d'objet comprennent une ou plusieurs des éléments suivants : accélération angulaire, vitesse ; et accélération.

13. Procédé selon l'une quelconque des revendications 10 à 12, comprenant la sortie d'un signal de commande en fonction de la corrélation déterminée entre la signature de suivi oculaire avec la signature de suivi d'objet.

14. Procédé selon l'une quelconque des revendications 10 à 13, dans lequel les informations de suivi d'objet comprennent un premier signal reçu à partir d'au moins un premier capteur.

15. Procédé selon l'une quelconque des revendications 10 à 14, dans lequel les informations de suivi oculaire comprennent un second signal reçu d'au moins un second capteur.
